# EUROPEAN PATENT SPECIFICATION

(11) **EP 0 613 947 B1**
(45) Date of publication and mention of the grant of the patent: **03.03.2004**
(21) Application number: 94101386.4
(22) Date of filing: 31.01.1994
(51) Int. Cl.: C12N 15/31, C12N 15/74, C12N 1/21, C07K 14/00, C12Q 1/68, C12P 21/08

(54) **Mitogenic factor, gene thereof and method of microdetection therefor**
Mitogener Faktor, sein Gen und Verfahren zur dessen Mikrodetektion
Facteur mitogénique, son gène et méthode pour sa microdetection

(30) Priority: 01.02.1993 JP 3738393
(43) Date of publication of application: 07.09.1994
(73) Proprietor: Beckman-Coulter, Inc., Fullerton, CA 92834 (US)
(72) Inventor: Yutsudo, Takashi, Kobe-shi, Hyogo-ken (JP); Okumura, Koichi, Takatsuki-shi, Osaka (JP); Iwasaki, Makoto, Amagasaki-shi, Hyogo-ken (JP); Hara, Ayako, Osaka-shi, Osaka 533 (JP); Kishishita, Masamichi, Kyoto-shi, Kyoto (JP); Takeda, Yoshifumi, dori, Kamigyo-ku, Kyoto-shi, Kyoto (JP); Igarashi, Hisanaga, Osaka-shi, Osaka (JP); Hinuma, Yorio, Kyoto-shi, Kyoto (JP)
(74) Representative: MICHELI & CIE

(56) References cited:
- EP-A- 0 266 686
- FEBS LETTERS, vol.308, no.1, August 1992 pages 30 - 34 YUTSUDO ET AL. 'A new type of mitogenic factor produced by Streptococcus pyogenes'
- FEBS LETTERS, vol.331, no.1,2, September 1993 pages 187 - 192 IWASAKI ET AL. 'Cloning, characterization and overexpression of a Streptococcus pyogenes gene encoding a new type of mitogenic factor'
- INFECT. IMMUN., vol.62, no.9, September 1994 pages 4000 - 4004 YUTSUDO ET AL. 'The gene encoding a new mitogenic factor in a Streptococcus pyogenes strain is distributed only in group A Streptococci'
- JAPANESE CIRCULATION JOURNAL, vol.48, no.12, December 1984 pages 1338 - 1342 MIYAKAWA ET AL. 'Comparative study of Streptococcal DNase by rapid and quantitative assay systems'
- DATABASE WPI Week 8629, Derwent Publications Ltd., London, GB; AN 86-185051 & JP-A-61 116 659 (TOYO JOZO KK) 4 June 1986

(1) GENERAL INFORMATION:
   (i) APPLICANT:
      (A) NAME: Shionogi & Co., Ltd.
      (B) STREET: 1-8, Doshomachi 3-chome, Chuo-ku
      (C) CITY: Osaka-shi, Osaka
      (E) COUNTRY: Japan
      (F) POSTAL CODE (ZIP): none
   (ii) TITLE OF INVENTION: MITOGENIC FACTOR, GENE THEREOF AND METHOD OF MICRODETECTION THEREFOR
   (iii) NUMBER OF SEQUENCES: 16
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: EP 94 10 1386.4
   (vi) PRIOR APPLICATION DATA:
      (A) APPLICATION NUMBER: JP 5-037383
      (B) FILING DATE: 01-FEB-1993
(2) INFORMATION FOR SEQ ID NO: 1:
   (i) SEQUENCE CHARACTERISTICS:
      (A) LENGTH: 1021 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: Streptococcus pyogenes
      (B) STRAIN: NY-5
   (ix) FEATURE:
      (A) NAME/KEY: CDS
      (B) LOCATION: 155..967
   (xi) SEQUENCE DESCRIPTION: SEQ ID NO: 1:
   ATGATATTAG TATGGCAAAA CAAAAAGACA ATGCCTTCTT TTTTCTCCTT ATTATCTCGT     60
   TTAATTTTCA TATTTTTTAA AAAAACTATT GATAAACTAG TTAAGTAAGC GTATACTATG    120
   GTTAGTGAGC GAAATTAGAA AAGAGGACAA GCAT ATG AAT CTA CTT GGA TCA         172
                                         Met Asn Leu Leu Gly Ser
                                          1               5
   AGA CGG GTT TTT TCT AAA AAA TGT CGG CTA GTG AAA TTT TCA ATG GTA       220
   Arg Arg Val Phe Ser Lys Lys Cys Arg Leu Val Lys Phe Ser Met Val
               10                  15                  20
   GCT CTT GTA TCA GCC ACA ATG GCT GTA ACA ACA GTC ACA CTT GAA AAT       268
   Ala Leu Val Ser Ala Thr Met Ala Val Thr Thr Val Thr Leu Glu Asn
           25                  30                  35
   ACT GCA CTG GCA CGA CAA ACA CAG GTC TCA AAT GAT GTT GTT CTA AAT       316
   Thr Ala Leu Ala Arg Gln Thr Gln Val Ser Asn Asp Val Val Leu Asn
       40                  45                  50
   GAT GGC GCA ACG AAG TAC CTA AAC GAA GCA TTA GCT TGG ACA TTC AAT       364
   Asp Gly Ala Thr Lys Tyr Leu Asn Glu Ala Leu Ala Trp Thr Phe Asn
   55                  60                  65                  70
   GAC AGT CCC AAC TAT TAC AAA ACC TTA GGT ACT AGT CAG ATT ACT CCA       412
   Asp Ser Pro Asn Tyr Tyr Lys Thr Leu Gly Thr Ser Gln Ile Thr Pro
                   75                  80                  85
   GCA CTC TTT CCT AAA GCA GGA GAT ATT CTC TAT AGC AAA TTA GAT GAG       460
   Ala Leu Phe Pro Lys Ala Gly Asp Ile Leu Tyr Ser Lys Leu Asp Glu
               90                  95                  100
   TTA GGA AGG ACG CGT ACT GCT AGA GGT ACA TTG ACT TAT GCC AAT GTT       508
   Leu Gly Arg Thr Arg Thr Ala Arg Gly Thr Leu Thr Tyr Ala Asn Val
           105                 110                 115
   GAA GGT AGC TAC GGT GTT AGA CAA TCT TTC GGT AAA AAT CAA AAC CCC       556
   Glu Gly Ser Tyr Gly Val Arg Gln Ser Phe Gly Lys Asn Gln Asn Pro
       120                 125                 130
   GCA GGA TGG ACT GGA AAC CCT AAT CAT GTC AAA TAT AAA ATT GAA TGG       604
   Ala Gly Trp Thr Gly Asn Pro Asn His Val Lys Tyr Lys Ile Glu Trp
   135                 140                 145                 150
   TTA AAT GGT CTG TCT TAT GTC GGA GAT TTC TGG AAT AGA AGT CAT CTC       652
   Leu Asn Gly Leu Ser Tyr Val Gly Asp Phe Trp Asn Arg Ser His Leu
                   155                 160                 165
   ATT GCA GAT AGT CTC GGT GGA GAT GCA CTC AGA GTC AAT GCC GTT ACA       700
   Ile Ala Asp Ser Leu Gly Gly Asp Ala Leu Arg Val Asn Ala Val Thr
               170                 175                 180
   GGG ACA CGT ACC CAA AAT GTA GGA GGT CGT GAC CAA AAA GGC GGC ATG       748
   Gly Thr Arg Thr Gln Asn Val Gly Gly Arg Asp Gln Lys Gly Gly Met
           185                 190                 195
   CGC TAT ACC GAA CAA AGA GCT CAA GAA TGG TTA GAA GCA AAT CGT GAT       796
   Arg Tyr Thr Glu Gln Arg Ala Gln Glu Trp Leu Glu Ala Asn Arg Asp
       200                 205                 210
   GGC TAT CTT TAT TAT GAA GCC GCT CCA ATC TAC AAC GCA GAC GAG TTG       844
   Gly Tyr Leu Tyr Tyr Glu Ala Ala Pro Ile Tyr Asn Ala Asp Glu Leu
   215                 220                 225                 230
   ATT CCA AGA GCT GTC GTG GTA TCA ATG CAA TCT TCT GAT AAT ACT ATC          892
   Ile Pro Arg Ala Val Val Val Ser Met Gln Ser Ser Asp Asn Thr Ile
                   235                 240                 245
   AAC GAG AAA GTA TTA GTT TAC AAC ACA GCT AAT GGC TAC ACC ATT AAT          940
   Asn Glu Lys Val Leu Val Tyr Asn Thr Ala Asn Gly Tyr Thr Ile Asn
               250                 255                 260
   TAC CAT AAC GGT ACA CCT ACT CAA AAA TAATACCAAA AGGCTAGACC                987
   Tyr His Asn Gly Thr Pro Thr Gln Lys
           265                 270
   TCTGCTCACT AGGCTAGCTT TTTACATCAA AAAA                                   1021
(2) INFORMATION FOR SEQ ID NO: 2:
   (i) SEQUENCE CHARACTERISTICS:
      (A) LENGTH: 271 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Asn Leu Leu Gly Ser Arg Arg Val Phe Ser Lys Lys Cys Arg Leu
    1               5                  10                  15
   Val Lys Phe Ser Met Val Ala Leu Val Ser Ala Thr Met Ala Val Thr
               20                  25                  30
   Thr Val Thr Leu Glu Asn Thr Ala Leu Ala Arg Gln Thr Gln Val Ser
           35                  40                  45
   Asn Asp Val Val Leu Asn Asp Gly Ala Thr Lys Tyr Leu Asn Glu Ala
       50                  55                  60
   Leu Ala Trp Thr Phe Asn Asp Ser Pro Asn Tyr Tyr Lys Thr Leu Gly
   65                  70                  75                  80
   Thr Ser Gln Ile Thr Pro Ala Leu Phe Pro Lys Ala Gly Asp Ile Leu
                   85                  90                  95
   Tyr Ser Lys Leu Asp Glu Leu Gly Arg Thr Arg Thr Ala Arg Gly Thr
               100                 105                 110
   Leu Thr Tyr Ala Asn Val Glu Gly Ser Tyr Gly Val Arg Gln Ser Phe
           115                 120                 125
   Gly Lys Asn Gln Asn Pro Ala Gly Trp Thr Gly Asn Pro Asn His Val
       130                 135                 140
   Lys Tyr Lys Ile Glu Trp Leu Asn Gly Leu Ser Tyr Val Gly Asp Phe
   145                 150                 155                 160
   Trp Asn Arg Ser His Leu Ile Ala Asp Ser Leu Gly Gly Asp Ala Leu
                   165                 170                 175
   Arg Val Asn Ala Val Thr Gly Thr Arg Thr Gln Asn Val Gly Gly Arg
               180                 185                 190
   Asp Gln Lys Gly Gly Met Arg Tyr Thr Glu Gln Arg Ala Gln Glu Trp
           195                 200                 205
   Leu Glu Ala Asn Arg Asp Gly Tyr Leu Tyr Tyr Glu Ala Ala Pro Ile
       210                 215                 220
   Tyr Asn Ala Asp Glu Leu Ile Pro Arg Ala Val Val Val Ser Met Gln
   225                 230                 235                 240
   Ser Ser Asp Asn Thr Ile Asn Glu Lys Val Leu Val Tyr Asn Thr Ala
                   245                 250                 255
   Asn Gly Tyr Thr Ile Asn Tyr His Asn Gly Thr Pro Thr Gln Lys
               260                 265                 270
(2) INFORMATION FOR SEQ ID NO: 3:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO: 3:
   ATGAATCTAC TTGGATCAAG A 21
(2) INFORMATION FOR SEQ ID NO: 4:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 4:
   GAGTAGGTGT ACCGTTATGG 20
(2) INFORMATION FOR SEQ ID NO: 5:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 5:
   TCGAATTCCA GGTCTCAAAT 20
(2) INFORMATION FOR SEQ ID NO: 6:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 6:
   CTAAGCTTCC TCGTTTAGGT 20
(2) INFORMATION FOR SEQ ID NO: 7:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (xi) SEQUENCE DESCRIPTION: SEQ ID NO: 7:
   AGAACACAAT ATACTGAATC AATGGT 26
(2) INFORMATION FOR SEQ ID NO: 8:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO: 8:
   ACTTTTCGCC ACCATTCCCA AGC 23
(2) INFORMATION FOR SEQ ID NO: 9:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 9:
   GCTCAACAAG ACCCCGATCC 20
(2) INFORMATION FOR SEQ ID NO: 10:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO: 10:
   TGATAGGCTT TGGATACCAT CG 22
(2) INFORMATION FOR SEQ ID NO: 11:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO: 11:
   GATCAAAACT TTGCTCGTAA CG 22
(2) INFORMATION FOR SEQ ID NO: 12:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO: 12:
   AGGTTTGATG CCTACAACAG C 21
(2) INFORMATION FOR SEQ ID NO: 13:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO: 13:
   GACTCTAAGA AAGACATTT 19
(2) INFORMATION FOR SEQ ID NO: 14:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO: 14:
   TTTTTCAAGA TAAATATCGA A 21
(2) INFORMATION FOR SEQ ID NO: 15:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (xi) SEQUENCE DESCRIPTION: SEQ ID NO: 15:
   ATTAACCCTC ACTAAAG 17
(2) INFORMATION FOR SEQ ID NO: 16:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (xi) SEQUENCE DESCRIPTION: SEQ ID NO: 16:
   AATACGACTC ACTATAG 17

## Description

The present invention relates to a mitogenic factor, a gene encoding the mitogenic factor, recombinant vectors containing said gene and transformed host cells, oligonucleotides for detecting the gene, a method of microdetection for the gene, a method of microdetection for group A streptococci, and antibodies which specifically recognise said factor.

The group A streptococci produce a number of extracellular proteins, among which are included the erythrogenic toxins. All substances which cause a skin reaction were originally defined as erythrogenic toxins, regardless of their biological activity or biochemical nature. The erythrogenic toxins are also known as scarlet fever toxin, streptococcal pyrogenic exotoxin (spe), streptococcal exotoxin, blastogens, and mitogens.

Three antigenically distinct erythrogenic toxins have been described, including type A, B, and C toxins. The most extensively studied toxin is the type A toxin, which was originally thought to be the toxin responsible for scarlet fever. The type A toxin is encoded by the bacteriophage speA gene and has a molecular weight of 25,787. The type B toxin is encoded by the bacteriophage speB gene and has a molecular weight of 27,588. The type C toxin is encoded by the bacteriophage speC gene and has a molecular weight of 24,354.

The erythrogenic toxins have been associated with a variety of biological activities, including the following: erythematous skin reactions, probably due to enhancement of delayed hypersensitivity; pyrogenicity; specific and nonspecific T-cell mitogenicity; immunosuppression by nonspecific activation of T-suppressor lymphocytes; enhancement of susceptibility to endotoxin shock; cytotoxicity to splenic macrophages; cardiotoxicity; alteration of the blood-brain barrier; and alteration of antibody response to sheep erythrocytes in mice and rabbits.

In view of the fact that at least two of the toxin genes, speA and speC, are located on mobile genetic elements, the frequency of occurrence of the erythrogenic toxin genes in a population of clinical isolates has been studied. According to these results, the speA gene was found in 15% of general strains and 45% of strains obtained from patients with scarlet fever (Yu C.E. and J.J. Ferreti, Infection and Immunity, 57, 3715 (1989)) and the speB gene was found in all group A strains tested and the speC gene was found in 50% of each of the general strains, isolates from patients with scarlet fever, and isolates from patients with rheumatic fever(Yu, C.E. et al., Infection and Immunity, 59, 211 (1991)).

The streptococci, especially strongly pathogenic group A streptococci produce streptolysin O (slo), a toxin responsible for beta hemolysis. In recovering patients infected with group A streptococci, rise of the antibody titer to the hemolytic toxin is observed. The determination of anti-streptolysin O antibody titer is now utilized for diagnosis of infectious disease caused by hemolytic streptococci.

However, any satisfactory method to detect a trace amount of cells of strongly pathogenic group A streptococci in early stages of infection has not yet been established, though such a method would be very helpful to the treatment of the infections.

In recent years, the present inventors have found that group A streptococci produce a new protein (a mitogenic factor) having a mitogen activity (Yutsudo, T., et al., Federation of European Biochemical Society Letters, 308, 30 (1992)).

The present inventors identified the gene encoding the mitogenic factor. Based upon this information, the present inventors have studied the distribution of the mitogenic factor gene among various streptococcal strains and found a strong association of the mitogenic factor gene with the group A streptococcal strains. Thus, the present inventors have established a method of microdetection for the gene. Using this method, a microdetection method for group A streptococci has also been established.

Accordingly, it is an object of the present invention to provide a nucleotide sequence encoding a mitogenic factor as defined in claim 1.

The term "hybridizing to said nucleotide sequence (a)", as used herein, refers to hybridizations at any stringency suitable to allow the successful hybridization, and optionally, the subsequent identification of a hybridizing DNA sequence. A general outline of suitable hybridization conditions is given, for example, in Maniatis et al., "Molecular Cloning, A Laboratory Manual", Cold Spring Harbor Laboratory (1989), 2nd Edition.

The term "nucleotide sequence which is degenerate as a result of the genetic code", refers to DNA sequences which code for the same amino acid sequence, but have a variant DNA sequence due to the degeneracy of the genetic code.

The invention further relates to recombinant vectors comprising the nucleotide sequence of the invention, preferably in functional association with an expression control sequence.

The vectors described herein, may be synthesized by techniques well known to the person skilled in the art. The components of the vectors, e.g. replicons, selection genes, and, with respect to expression control sequences, promoters, enhancers and ribosome binding sites, and the like, may be obtained from natural sources or synthesized by known procedures.

For example, when using E. coli as host, plasmid pBR322 can be generally employed. Examples of promoters usable for expression are the tryptophan promoter, P_{L} promoter, lac promoter, lpp promoter, etc. A suitable expression vector for the transformation of a suitable microbial strain such as E. coli JM 109 (Takara Shuzo Co., Ltd) is, for example, pSPORT 1 (BRL Company). For the production of the mitogenic factor encoded by the nucleotide sequence of the invention, mammalian expression can also be used employing, for example,'pXM (Y.C. Yang et al., Cell 47 (1986), 3-10) which is a mammalian expression vector containing the SV40 origin of replication and enhancer.

The invention also relates to host cells which are transformed with the recombinant DNA molecules of the present invention by standard molecular biology techniques. Preferably such host organisms are bacteria, such as E. coli or Bacillus subtilis, yeast, such as species or strains of Saccharomyces (e.g. Saccharomyces cerevisiae), and other eukaryotic host cells, such as plant, insect or mammalian cells, e.g. COS-1, 3T3 and CHO cells.

It is a further object of the present invention to provide a mitogenic factor encoded by the nucleotide sequence of the invention, as specified above.

Preferably, said mitogenic factor has the following amino acid sequence (SEQ ID NO: 2):

It is still a further object of the present invention to provide a method for the production of the mitogenic factor comprising culturing any of the transformed host cells mentioned above under conditions permitting transcription of the transcribable DNA and translation of the produced RNA into a polypeptide, and recovering said mitogenic factor from the culture.

It is another object of the present invention to provide a nucleotide sequence which is complementary to the nucleotide sequence encoding a protein having the biological activity of said mitogenic factor.

It is a further object of the present invention to provide an oligonucleotide having less than 100 bases, comprising a partial sequence of the nucleotide sequence of the present invention or of the complementary sequence, which can be hybridized with either of the nucleotide sequence.

It is a still further object of the present invention to provide a method of microdetection for the mitogenic factor gene, wherein a fragment of the mitogenic factor gene is amplified and accumulated by repeating the cycles of a polymerase chain reaction (PCR) with a polymerase, preferably a thermostable polymerase, using the two oligonucleotides of the present invention as the primers and the polynucleotides of the present invention as the template DNA, and wherein the amplified and accumulated fragments of the mitogenic factor gene are detected.

It is another object of the present invention to provide a method of microdetection for group A streptococci, wherein the mitogenic factor gene is detected by the method of the present invention.

A further object of the invention is to provide antibodies, preferably monoclonal antibodies, which are capable of specifically recognising and binding to the mitogenic factor of the invention.

The term "antibody" relates to any antibody obtainable by immunization of an animal with the polypeptide of the invention as immunogen or hapten or by genetic engineering or by a combination thereof. Genetically engineered antibodies may be obtained, e.g. by expression in mammalian, bacterial, yeast or plant cells. Depending upon the host cell, the glycosylation pattern of said antibody will vary. Genetically engineered antibodies also comprise humanised antibodies with at least a part of the variable region derived from human genes and the remainder derived from one or different other organisms.

Methods for producing said polyclonal or monoclonal antibodies are well known in the art and have been described in detail, e.g. in Harlow, E. and Lane, D., "Antibodies - A Laboratory Manual", Cold Spring Harbor Laboratory (1988).

The present invention makes it possible to detect strongly pathogenic group A streptococci in an early stage of the infection, and thus can be utilized for an early diagnosis of the infectious disease with the bacteria.

As used herein, the mitogenic factor of the present invention has an amino acid sequence (SEQ ID NO:2) deduced from the entire nucleotide sequence obtained in an example given below. The mitogenic factor of the present invention, a thermostable polypeptide containing the entire amino acid sequence, exhibits rabbit peripheral blood lymphocyte mitogenicity and/or DNA hydrolyzing activity. It will be understood that natural variations exist and occur among the bacteria. These variations may be demonstrated by amino acid difference in the overall sequence or by deletions, substitutions or insertions of amino acids in the sequence. All such variations are included within the scope of this invention so long as the essential, characteristic activity of the mitogenic factor remains unaffected in kind.

Nucleotide sequences comprising the nucleotide sequence encoding the mitogenic factor of the present invention include all nucleotide sequences by which a polypeptide possessing mitogenic factor activity such as rabbit peripheral blood lymphocyte mitogenicity and/or DNA hydrolyzing activity can be expressed in a conventional, suitable host-vector system. Because an amino acid is represented by several combinations of three bases (triplet code) in many cases, the nucleotide sequences of the present invention include all nucleotide sequences corresponding to the same polypeptide, exemplified by the sequence represented by SEQ ID NO:2. Nucleotide sequences comprising a nucleotide sequence complementary to that encoding the mitogenic factor include all nucleotide sequences comprising a nucleotide sequence complementary to that of any one of the above-described nucleotide sequences.

An oligonucleotide of the present invention which comprises less than 100 bases is capable of hybridizing with either a nucleotide sequence encoding the mitogenic factor or a nucleotide sequence complementary thereto. It comprises a partial sequence of the nucleotide sequence comprising the nucleotide sequence encoding the mitogenic factor or the nucleotide sequence complementary thereto. These oligonucleotides are useful as primers for amplifying mitogenic factor gene fragments by the PCR method. The oligonucleotide of the present invention comprising a partial sequence of the nucleotide sequence comprising the nucleotide sequence encoding the mitogenic factor or the nucleotide sequence complementary thereto can hybridize with a nucleotide sequence complementary thereto. It also hybridizes with the gene encoding the mitogenic factor even when it has an additional oligonucleotide sequence bound thereto. Such oligonucleotides having an additional oligonucleotide sequence bound thereto and capable of hybridization are also included within the scope of the present invention. Also, since the efficiency of oligonucleotide synthesis lowers as the number of bases exceeds 100, those having more than 100 bases are unnecessary for practical use. Usually, the number of bases is preferably 16 to 36, since the desired specific reaction for PCR can be achieved within this range.

The PCR method is a conventional method for amplifying and accumulating a DNA fragment of a given length by repeating the cycle of denaturation of DNA, primer annealing and extension, using preferably a thermostable DNA-dependent DNA polymerase which does not lose its activity even at the melting point of the DNA, a template DNA and a primer capable of binding to the template. Specifically, the DNA is denatured at a high temperature of not less than 90°C, then anneals to the primer at a low temperature of from 25 to 70°C, followed by extension with the polymerase.

The oligonucleotide used as a primer to amplify a part of a mitogenic factor gene by the PCR method is therefore required to be capable of hybridizing with a portion near the 3'-end of the sense or antisense chain of the denatured mitogenic factor gene. This is because identification and detection of the mitogenic factor gene can be increasingly facilitated and assured when the amplified DNA has a size similar to the size of the open reading flame (ORF) of the mitogenic factor. However, mutual binding of the oligonucleotide selected must be avoided.

Specifically, for example, when an oligonucleotide sequence (a) of 5'-ATGAATCTACTTGGATCAAGA-3'(SEQ ID NO: 3) chosen from the vicinity of 5'-end of the mitogenic factor gene and an oligonucleotide sequence (b) of 5'-GAGTAGGTGTACCGTTATGG-3' (SEQ ID NO: 4) chosen from the vicinity of 5'-end of the complementary sequence are used as the PCR primers, the mitogenic factor gene can be amplified and detected as a band of 808 bp as shown in the Examples below.

The above-described oligonucleotide functions well as a primer to amplify a mitogenic factor gene by the PCR method even when it has one or more bases deleted therefrom or added thereto, as long as it retains its capability of hybridizing with the mitogenic factor gene. Specifically, in the case of the above-described oligonucleotide sequences (a) and (b) consisting of 21 and 20 bases, respectively, at least 16 consecutive bases in these sequences are sufficient to retain their primer function, and the total number of bases is preferably not more than 36 when additional bases are added to the sequence (a) or (b). Here, the polynucleotide of the present invention is used as a template DNA. Specifically, the DNA extracted from group A streptococci is used therefor. The procedures and conditions for the PCR are not limitative and optionally selected from the conventional method.

Microdetection of a mitogenic factor gene by the PCR method is possible using the above-described oligonucleotides as primers, representing the gist of the present invention. Usually, it is absolutely impossible to detect the target gene in a small number of cells. However, when PCR is performed using the above-described oligonucleotides as primers, a mitogenic factor gene can be detected from only a small number of cells because the target portion of the mitogenic factor gene is selectively amplified several hundred million folds for normally about 30 cycles. In other words, when a small number of cells of a bacterium producing a mitogenic factor are obtained from a pathological material, they can be directly used as a sample for detection of the mitogenic factor gene without cultivation. The detection of such amplified and accumulated DNA can be carried out by conventional methods such as agarose gel electrophoresis, ethidium bromide staining and the Southern hybridization analysis.

Using the above-described method for detecting the mitogenic factor gene, the distribution of the mitogenic factor gene among various bacterial strains is studied in the present invention. The test bacterium is cultured as described below in Examples, and a given amount of the bacterial culture is collected and heated, followed by the PCR in a PCR buffer in the presence of dNTP and Taq polymerase (produced by Takara Shuzo Co., Ltd.). The sample thus treated is subjected to the Southern hybridization analysis, and analyzed for the 808-bp polynucleotide. About 400 strains of various streptococcal bacteria obtained from hospitals etc., as test bacteria, are analyzed for the occurrence of the mitogenic factor gene to know the distribution of the gene among the bacterial strains by the detection method of the present invention. As shown in the Examples, 371 strains except one strain of group A streptococci were found to have a mitogenic factor gene. The only exceptional strain has neither the spe gene nor the slo gene. With respect to non-group A streptococci, no strain is found to be positive for the mitogenic factor gene. Thus, group A streptococci can be specifically detected by the presence of the mitogenic factor gene. The method of microdetection for the mitogenic factor gene of the present invention can therefore serve excellently as a method of microdetection for group A streptococci. From the results of the experiments conducted together with the above experiments, it is evident that the microdetection of the mitogenic factor gene in the present invention serves the most excellent method in specificity to group A streptococci, in comparison with the detection of the spe gene and the slo gene. The method of microdetection of the present invention seems to be useful for diagnosis in an early stage of infectious disease with group A streptococci because it can detect a mitogenic factor gene even when only several cells are available.

When a bacterium producing a mitogenic factor is used as the test bacterium in the present microdetection method, the amount of DNA fragments amplified by the PCR method may decrease over time due to digestion because the mitogenic factor itself possesses thermostable DNase activity. Therefore, when the microdetection method of the present invention is used, the DNase activity of the mitogenic factor may be previously inactivated considering the possibility that it may take a long time from the PCR amplification to the Southern hybridization analysis. As a means for the pre-treatment of the inactivation, thermal inactivation may be considered, but it does not seem to be effective because the DNase is highly thermostable. With this in mind, the inactivating effects of various proteases are examined. As a result, pre-treatment with such as proteinase K and trypsin are found to be effective. The amount of such proteinase is normally adjusted to a final concentration of 5 µg/ml and the pre-treatment is carried out at 37°C for 30 minutes. Preferably, such treatment is carried out by, for example, treating the bacterial solution with a proteinase before or after thermal extraction of DNA and then inactivating the protease by 5-minute incubation at 100°C.

### EXAMPLES

The present invention is hereinafter described in more details by means of the following working examples, but the present invention is not limited by them.

The strains used in Examples and Comparative Example were 371 strains of *Streptococcus pyogenes*, 15 strains of non-group A streptococci and 11 strains of various other bacteria. The standard strain of *Streptococcus pyogenes* was NY-5, obtained from Saitama College of Health.

### Example 1: Determination of nucleotide sequence of mitogenic factor gene

On the basis of the N-terminal sequence of 21 amino acids of the mitogenic factor reported by the present inventors [Yutsudo, T. et al., FEBS LETT., 308, 30 (1992)], mixed PCR primers of the nucleotide sequences deduced from it were synthesized for amplification of a DNA corresponding to the sequence of the 21 amino acids. The PCR was carried out using these primers, and the nucleotide sequence of the DNA fragment thus amplified was determined using dsDNA Cyclic Sequencing System (manufactured by GIBCO BRL, Life Technologies, Inc.).

On the basis of this nucleotide sequence, the following primers for obtaining the full-length mitogenic factor gene were newly synthesized: 71-bp fragment was amplified by the PCR using these primers.

On the other hand, since the mitogenic factor gene was found to be contained in the 2.1 kb PstI-HindIII fragment by the Southern hybridization analysis of the genomic DNA, the genomic DNA derived from NY-5 strain was digested to completion with PstI and HindIII and electrophoresed. After the electrophoresis, a fragment corresponding to 2.1 kb in size was recovered from the gel piece and ligated to a vector (pBluescript, manufactured by Stratagene).

Using this as a template DNA, the PCR was performed using the primers on the vector side (5'-ATTAACCCTCACTAAAG-3' (SEQ ID NO: 15), 5'-AATACGACTCACTATAG-3' (SEQ ID NO: 16), and the above-described primers (SEQ ID NO:5, SEQ ID NO:6) to yield a fragment containing the mitogenic factor gene. This fragment was analyzed by the Sanger method [Proc. Natl. Acad. Sci., 74, 5463 (1977)] to determine its entire nucleotide sequence. On the basis of this nucleotide sequence, the amino acid sequence of SEQ ID NO:1 was deduced.

### Example 2: Preparation of primers

An oligonucleotide (a) consisting of 21 bases, 5'-ATGAATCTACTTGGATCAAGA-3' (SEQ ID NO:3), chosen from the vicinity of the 5'-end of the mitogenic factor gene, and another oligonucleotide (b) consisting of 20 bases, 5'-GAGTAGGTGTACCGTTATGG-3' (SEQ ID NO:4), chosen from the vicinity of the 5'-end of the complementary sequence, were chemically synthesized in the presence of the β-link•beta cyanoethyl phosphoamidite reagent(manufactured by Milligen/Biosearch Company) using Cyclone, a DNA synthesizer(manufactured by Milligen/Biosearch Company). The synthesized oligonucleotides were purified by HPLC using a reversed-phase column (C18) to yield the desired primers.

These primers (primer (a) and primer (b), respectively) serve excellently for amplification of mitogenic factor gene because they do not bind mutually and lack homology to other genes.

### Example 3: Amplification of mitogenic factor gene fragment by the PCR

Each bacterial strain was subjected to overnight shaking culture at 37°C in BHI broth (brain heart infusion medium, manufactured by Difco) and then diluted 10 folds with TE buffer (10 mM Tris-HCl, 1 mM EDTA, pH 7.5). Each diluted bacterial solution was heated at 100°C for 5 minutes.

A 2 µl aliquot of this treated bacterial solution was adjusted to 50 µl with 5 µl of 10 x PCR buffer (100 mM Tris-HCl, pH 8.3, 500 mM KCl, 15 mM MgCl₂, 0.1% gelatin), 8 µl of dNTP, 0.25 µl of 5 unit/ml Taq DNA polymerase (manufactured by Takara Shuzo Co., Ltd.), 0.5 µl of each of 10 pmol primers (a) and (b) and sterile distilled water.

The PCR was performed using thermal sequencer TSR-300 (produced by Iwaki Glass Co., Ltd.), in which thermal denaturation of DNA at 94°C for 40 seconds, annealing at 55°C for 90 seconds and chain extension at 72°C for 60 seconds were repeated in 30 cycles, followed by a reaction at 72°C for 60 seconds. The amplified DNA was subjected to 2% agarose gel electrophoresis and detected by ethidium bromide staining. For confirmation, the amplified DNA was also subjected to the following Southern hybridization. Specifically, after the PCR-treated solution was subjected to agarose gel electrophoresis, the gel was transferred to a Hybond-N⁺ membrane (manufactured by Amersham Japan, Ltd.) in alkaline solution by a conventional method. The probe was prepared by labeling the DNA fragment of the mitogenic factor derived from NY-5 strain, which had been amplified by the PCR, with ³²P using Multiprime DNA Labeling System (manufactured by Amersham Japan, Ltd.) after the nucleotide sequence was confirmed by direct sequencing.

Using a hybridization mixture of 6 x SSC (1 x SSC is composed of 0.15 M NaCl and 0.015 M sodium citrate), 5 x Denhardt's solution, 0.5% SDS, and 0.5 mg/ml heparin, overnight hybridization was conducted at 65°C. The filter was washed in 2 x SSC and 0.5% SDS at a room temperature for 5 minutes, then twice in 1 x SSC and 0.5% SDS at 42°C for 15 minutes, and then in 0.1 x SSC and 0.5% SDS at 42°C for 1 hour, after which genes were detected using a bioimage analyzer FUJIX BA100 (manufactured by Fuji Photo Film Co., Ltd.) and on the films developed. The results are given in Tables 1 through 4.

**Table 1**

| Detection of mf(mitogenic factor) gene and slo gene by the PCR method | | |
|---|---|---|
| | mf+ | mf- |
| slo+ | 370 | 0 |
| slo- | 0 | 1 |

**Table 2**

| Comparison in number between mf gene positive and spe gene positive strains | | | | |
|---|---|---|---|---|
| | speA+ speC+ | speA+ speC- | speA- speC+ | speA- speC- |
| mf+ speB+ | 19 | 27 | 245 | 76 |
| mf+ speB- | 0 | 2 | 0 | 1 |
| mf- speB+ | 0 | 0 | 0 | 0 |
| mf- speB- | 0 | 0 | 0 | 1* |

| | | | | |
|---|---|---|---|---|
| * : slo- | | | | |

**Table 3**

| Detection of mf gene and slo gene in non-group A streptococci | | | |
|---|---|---|---|
| Sample No. | | slo | mf |
| 381 | B(Str. agalactiae) | - | - |
| 382 | B(Str. agalactiae) | - | - |
| 384 | B(Str. agalactiae) | - | - |
| 386 | B(Str. agalactiae) | - | - |
| 387 | B(Str. agalactiae) | - | - |
| 388 | B(Str. agalactiae) | - | - |
| 42 | C | + | - |
| 43 | C | + | - |
| 44 | C | + | - |
| 383 | C | + | - |
| 92 | G | + | - |
| 385 | G | + | - |
| 389 | G | + | - |
| 390 | G | + | - |
| 391 | G | + | - |

**Table 4**

| Distribution of mf gene and slo gene among various bacterial strains | | | |
|---|---|---|---|
| Strain | | slo | mf |
| Bacillus cereus | RIMD 0206001 | + ¹⁾ | - |
| Corynebacterium diphtheriae | RIMD 0343044 | - | - |
| Microccus luteus | RIMD 1303002 | - | - |
| Leuconostoc mesenteroides | RIMD 1204002 | - | - |
| Staphylococcus aureus | RIMD 3109017 | - | - |
| Streptococcus faecalis | RIMD 3116001 | + ²⁾ | - |
| Streptococcus mutans | RIMD 3125001 | - | - |
| Streptococcus sanguis | SSH 83(MCLS-1) | - | - |
| Streptococcus sanguis | KIH T(MCLS-2) | - | - |
| Pseudomonas aeruginosa | RIMD 1603002 | - | - |
| Salmonella enteritidis | RIMD 1933001 | - | - |

| | | | |
|---|---|---|---|
| 1): Two bands are seen, one having the same molecular weight as the band obtained from NY-5 strain; the other having a smaller molecular weight. | | | |
| 2): Two bands are seen, both having a smaller molecular weight than the band obtained from NY-5 strain. | | | |

### Comparative Example

To compare the methods of microdetection of group A streptococci, the distribution of the genes encoding other erythrogenic toxins produced by *Streptococcus pyogenes*, streptolysin O (slo) of a hemolytic toxin and streptococcal pyrogenic exotoxin (spe) were studied. Since the nucleotide sequences of these genes are known, their DNA fragments can be amplified by the PCR method using suitable primers.

As the primers to amplify the slo gene, the following oligonucleotides were prepared according to the known nucleotide sequence: 5'-AGAACACAATATACTGAATCAATGGT-3' (SEQ ID NO: 7) and 5'-ACTTTTCGCCACCATTCCCAAGC-3' (SEQ ID NO: 8). Using these primers, the 878-bp gene fragment can be amplified by the PCR method.

As for the spe gene, the nucleotide sequences have been determined for all three subtypes, speA, speB and speC. Based on the known nucleotide sequences, the following primers were prepared: and for speA; and for speB; and and for sepC.

Whenever the PCR was carried out using these primers, NY-5 strain was used as the positive control and *Escherichia coli* lacking these genes as the negative control. The slo gene was amplified by the PCR method in the same manner as in Example 3. The speA, speB and speC genes were amplified as follows: To a reaction mixture containing 10 mM Tris-HCl(pH 8.5), 50 mM KCl, 0.01% gelatin, 0.2 mM dNTP (dATP, dCTP, dTTP, dGTP) and 0.1 to 1 µM primers, 1 µl of a heated bacterial solution and 2.5 units of Taq DNA polymerase (produced by Perkin-Elmer Cetus) were added, and 50 µl of mineral oil was overlaid. The amplification reaction was carried out using a thermal sequencer TRS300 (produced by Iwaki Glass Co., Ltd.), in which thermal denaturation of DNA at 94°C for 1 minute, annealing at 52°C for 1.5 minutes and chain extension at 72°C for 1.5 minutes were repeated in 30 cycles.

The distributions of the thus-obtained slo, speA, speB and speC genes among the test bacterial strains are given in Tables 1 through 4. From Table 1, it is seen that all but one test strains of *Streptococcus pyogenes* (370 strains) had the mitogenic factor gene and the slo gene. As for the speA, speB and speC genes, 48 strains had the speA gene, 367 strains had the speB gene and 264 strains had the speC gene (Table 2). All strains having any one of the speA, speB and speC genes had the mitogenic factor gene (Table 2). On the other hand, all non-group A streptococci lacked the mitogenic factor gene, while the strains in groups C and G had the slo gene (Table 3). Table 4 shows the results on the distributions of the mitogenic factor gene and the slo gene among various bacterial strains other than group A streptococci. The mitogenic factor gene was not detected in any of the strains, while the slo gene was detected in 2 strains.

In summary, unlike the slo and spe genes, the distribution of the mitogenic factor gene is limited to group A streptococci. Therefore, the microdetection of the mitogenic factor in the present invention can be the preferred method for the microdetection of group A streptococci.

### Example 4

When the PCR is immediately followed by agarose gel electrophoresis in the detection of the mitogenic factor gene, a band of the amplified DNA fragment appears, but when electrophoresis is conducted after a long period of time, no bands are detected. This is because the mitogenic factor itself possesses DNase activity, by which the amplified DNA fragment is digested.

With this in mind, to inactivate the DNase activity of the mitogenic factor, the following treatments (1) to (4) were performed:
(1) Proteinase K or trypsin was added to the bacterial solution diluted 10 folds with TE buffer as described in Example 3 to a final concentration of 5 µg/ml, followed by incubation at 37°C for 30 minutes, after which the protease was inactivated by a heat treatment at 100°C for 5 minutes, and then PCR was carried out.
(2) The bacterial solution was heated at 100°C for 5 minutes and then subjected to the PCR.
(3) The bacterial solution was rapidly heated from -80°C to 100°C and then subjected to the PCR.
(4) The bacterial solution was centrifuged to remove the supernatant, after which the bacterial cells were subjected to the PCR.

As a result, even 48 hours after the PCR, a clear band appeared with the bacterial solution subjected to the proteinase K or trypsin treatment of (1) above and with the bacterial solution after the supernatant removal treatment of (4). This fact suggests that the mitogenic factor protein is stable to heat and solubilized extracellularly and undergoes digestion with trypsin or proteinase K.

In performing the microdetection method of the present invention, which is mainly intended to detect group A streptococci producing the mitogenic factor, it is preferred to have a pre-treatment of the bacterial sample suspension with a protease before the PCR amplification, in consideration of the possibility that pathological materials obtained from lesions may contain the mitogenic factor protein.
## Claims

1. A nucleotide sequence encoding a mitogenic factor selected from the group consisting of:
(a) the nucleotide sequence from position 155 to 967 of SEQ ID NO: 1;
(b) a nucleotide sequence hybridizing to said nucleotide sequence (a) under high stringency;
(c) a nucleotide sequence which is degenerate as a result of the genetic code; and
(d) a nucleotide sequence which is complementary to the nucleotide sequence of section (a), (b) or (c).

2. A recombinant vector comprising a nucleotide sequence of claims 1.

3. The recombinant vector of claim 2 wherein said nucleotide sequence is functionally associated with an expression control sequence.

4. A host cell containing the recombinant vector of claim 2 or 3.

5. The host cell of claim 4 which is a bacterial cell, a mammalian cell, an insect cell, a yeast cell or a plant cell.

6. A method for the production of the mitogenic factor encoded by the nucleotide sequence of claim 1 or the vector of claim 2 or 3, comprising cultivating a host cell of claim 4 or 5 and recovering said mitogenic factor from the culture.

7. An oligonucleotide with a length of 16 to 36 bases, consisting of a partial sequence of the nucleotide sequence of claim 1 (a), (b) or (c), which can be hybridized with the nucleotide sequence of claim 1 (d).

8. The oligonucleotide according to claim 7, which can be used as a primer for a polymerase chain reaction.

9. The oligonucleotide according to claim 8, wherein the sequence is sequence (a) (SEQ ID NO: 3): 5'-ATGAATCTACTTGGATCAAGA-3'.

10. The oligonucleotide according to claim 8, wherein said oligonucleotide comprises 16 to 36 bases, in which 16 or more consecutive bases in the sequence (a) of claim 9 are contained therein.

11. An oligonucleotide with a length of 16 to 36 bases, consisting of a partial sequence of the nucleotide sequence of claim 1 (d), which can be hybridized with the polynucleotide of claim 1 (a), (b) or (c).

12. The oligonucleotide according to claim 11, which can be used as a primer for a polymerase chain reaction.

13. The oligonucleotide according to claim 12, wherein the sequence is sequence (b) (SEQ ID NO: 4): 5'-GAGTAGGTGTACCGTTATGG-3'.

14. The oligonucleotide according to claim 13, wherein said oligonucleotide comprises 16 to 36 bases, in which 16 or more consecutive bases in the sequence (b) of claim 13 are contained therein.

15. A method of microdetection of the mitogenic factor gene, comprising the steps of amplifying and accumulating a fragment of a mitogenic factor gene contained in a sample by repeating the cycles of a polymerase chain reaction with a polymerase, preferably a thermostable polymerase, using the two oligonucleotides of claims 8 and 12 as the primers and detecting the amplified and accumulated fragment of the mitogenic factor gene.

16. The method according to claim 15, wherein one primer is the oligonucleotide of claims 9 or 10 and the other primer is the oligonucleotide of claims 13 or 14.

17. The method according to claim 15 or 16, wherein the primers have the sequences of SEQ ID NOs: 3 and 4, respectively.

18. The method according to any one of claims 15 to 17, wherein the nucleotide sequences which serve as the template DNA are extracted from group A streptococci.

19. A method of microdetection for group A streptococci, comprising the step of detecting the mitogenic factor gene by the method of any one of claims 15 to 18.

20. The method according to claim 19, further comprising the step of pre-treating the test bacteria with a protease to inactivate the DNAse activity.

## Patentansprüche

1. Eine einen mitogenen Faktor kodierende Nucleotidsequenz ausgewählt aus der Gruppe bestehend aus:
(a) der Nucleotidsequenz von Position 155 bis 967 der SEQ ID NR: 1;
(b) einer unter hoher Stringenz zu besagter Nucleotidsequenz (a) hybridisierenden Nucleotidsequenz;
(c) einer Nucleotidsequenz, welche als Ergebnis des genetischen Codes degeneriert ist; und
(d) einer Nucleotidsequenz, welche zu den Nucleotidsequenzen aus Sektion (a), (b) oder (c) komplementär ist.

2. Ein eine Nucleotidsequenz des Anspruchs 1 aufweisender rekombinanter Vektor.

3. Der rekombinante Vektor des Anspruchs 2, wobei die besagte Nucleotidsequenz funktional mit einer Expressionskontrollsequenz assoziiert ist.

4. Eine den rekombinanten Vektor des Anspruchs 2 oder 3 enthaltende Wirtszelle.

5. Die Wirtszelle des Anspruchs 4, welche eine Bakterienzelle, eine Säugetierzelle, eine Insektenzelle, eine Hefezelle oder eine Pflanzenzelle ist.

6. Ein Verfahren zur Herstellung des von der Nucleotidsequenz des Anspruchs 1 oder dem Vektor des Anspruchs 2 oder 3 kodierten mitogenen Faktors, das Kultivieren einer Wirtszelle des Anspruchs 4 oder 5 und die Rückgewinnung des besagten mitogenen Faktors von der Kultur aufweisend.

7. Ein Oligonucleotid mit einer Länge von 16 bis 36 Basen, bestehend aus einer Teilsequenz der Nucleotidsequenz des Anspruchs 1 (a), (b) oder (c), welche mit der Nucleotidsequenz des Anspruchs 1 (d) hybridisiert werden kann.

8. Das Oligonucleotid gemäß Anspruch 7, welches als Auslöser für eine Polymerase-Kettenreaktion benutzt werden kann.

9. Das Oligonucleotid gemäß Anspruch 8, wobei die Sequenz die Sequenz (a) (SEQ ID NR: 3): 5'-ATGAATCTACTTGGATCAAGA-3' ist.

10. Das Oligonucleotid gemäß Anspruch 8, wobei besagtes Oligonucleotid 16 bis 36 Basen aufweist, in dem 16 oder mehr konsekutive Basen in der Sequenz (a) des Anspruchs 9 enthalten sind.

11. Ein Oligonucleotid mit einer Länge von 16 bis 32 Basen, bestehend aus einer Teilsequenz der Nucleotidsequenz des Anspruchs 1 (d), welche mit dem Polynucleotid des Anspruchs 1 (a), (b) oder (c) hybridisiert werden kann.

12. Das Oligonucleotid gemäß Anspruch 11, welches als Auslöser für eine Polymerase-Kettenreaktion benutzt werden kann.

13. Das Oligonucleotid gemäß Anspruch 12, wobei die Sequenz die Sequenz (b) (SEQ ID NR: 4): 5'-GAGTAGGTGTACCGTTATGG-3' ist.

14. Das Oligonucleotid gemäß Anspruch 13, wobei besagtes Oligonucleotid 16 bis 36 Basen aufweist, in dem 16 oder mehr konsekutive Basen in der Sequenz (b) des Anspruchs 13 enthalten sind.

15. Ein Verfahren zur Mikrodetektion des mitogenen Faktorgens, die Schritte der Verstärkung und Anhäufung eines Fragments eines in einer Probe enthaltenen mitogenen Faktorgens durch Wiederholen der Zyklen einer Polymerase-Kettenreaktion mit einer Polymerase, bevorzugt einer thermostabilen Polymerase, aufweisend, wobei die beiden Oligonucleotide der Ansprüche 8 und 12 als Auslöser benutzt und die verstärkten und angehäuften Fragmente des mitogenen Faktorgens detektiert werden.

16. Das Verfahren gemäß Anspruch 15, wobei ein Auslöser das Oligonucleotid der Ansprüche 9 oder 10 und der andere Auslöser das Oligonucleotid der Ansprüche 13 oder 14 ist.

17. Das Verfahren gemäß Anspruch 15 oder 16, wobei die Auslöser die Sequenzen der SEQ ID NR: 3 beziehungsweise 4 aufweisen.

18. Das Verfahren gemäß einem der Ansprüche 15 bis 17, wobei die als DNA-Vorlage dienenden Nucleotidsequenzen aus den Gruppe A Streptococci extrahiert werden.

19. Ein Verfahren zur Mikrodetektion von Gruppe A Streptococci, den Schritt des Detektierens des mitogenen Faktrogens mittels des Verfahrens gemäß einem der Ansprüche 15 bis 18 aufweisend.

20. Das Verfahren gemäß Anspruch 19, weiterhin den Schritt des Vorbehandelns der Testbakterien mit einer Protease zur Inaktivierung der DNA-Aktivität aufweisend.

## Revendications

1. Séquence nucléotidique codant un facteur mitogène choisie dans le groupe constitué par :
(a) la séquence nucléotidique allant de la position 155 à la position 967 de SEQ ID NO: 1 ;
(b) une séquence nucléotidique s'hybridant dans des conditions de forte stringence avec ladite séquence nucléotidique (a) ;
(c) une séquence nucléotidique qui est dégénérée en conséquence du code génétique ; et
(d) une séquence nucléotidique qui est complémentaire de la séquence nucléotidique de la section (a), (b) ou (c).

2. Vecteur recombinant comprenant une séquence nucléotidique selon la revendication 1.

3. Vecteur recombinant selon la revendication 2, dans lequel ladite séquence nucléotidique est fonctionnellement associée à une séquence de commande d'expression.

4. Cellule hôte comprenant le vecteur recombinant selon la revendication 2 ou 3.

5. Cellule hôte selon la revendication 4, qui est une cellule bactérienne, une cellule mammifère, une cellule d'insecte, une cellule de levure ou une cellule de plante.

6. Procédé pour la production du facteur mitogène codé par la séquence nucléotidique de la revendication 1 ou le vecteur de la revendication 2 ou 3, comprenant la culture d'une cellule hôte selon la revendication 4 ou 5 et la récupération dudit facteur mitogène à partir de la culture.

7. Oligonucléotide ayant une longueur de 16 à 36 bases, consistant en une séquence partielle de la séquence nucléotidique de la revendication 1 (a), (b) ou (c), qui peut être hybridée avec la séquence nucléotidique de la revendication 1 (d).

8. Oligonucléotide selon la revendication 7, qui peut être utilisé comme amorce pour une réaction de polymérisation en chaîne.

9. Oligonucléotide selon la revendication 8, dans lequel la séquence est la séquence (a) (SEQ ID NO: 3): 5'-ATGAATCTACTTGGATCAAGA-3'.

10. Oligonucléotide selon la revendication 8, comprenant 16 à 36 bases, dans lesquelles sont comprises 16 ou plus de bases consécutives de la séquence (a) de la revendication 9.

11. Oligonucléotide ayant une longueur de 16 à 36 bases, consistant en une séquence partielle de la séquence nucléotidique de la revendication 1 (d), qui peut être hybridée avec le polynucléotide de la revendication 1 (a), (b) ou (c).

12. Oligonucléotide selon la revendication 11, qui peut être utilisé comme amorce pour une réaction de polymérisation en chaîne.

13. Oligonucléotide selon la revendication 12, dans lequel la séquence est la séquence (b) (SEQ ID NO: 4): 5'-GAGTAGGTGTACCGTTATGG-3'.

14. Oligonucléotide selon la revendication 13, comprenant 16 à 36 bases, dans lesquelles sont comprises 16 ou plus de bases consécutives de la séquence (b) de la revendication 13.

15. Procédé de microdétection du gène du facteur mitogène, comprenant les étapes consistant à amplifier et accumuler un fragment d'un gène de facteur mitogène contenu dans un échantillon en répétant les cycles d'une réaction de polymérisation en chaîne avec une polymérase, de préférence une polymérase thermostable, en utilisant les deux oligonucléotides des revendications 8 et 12 comme amorces, et détecter le fragment amplifié et accumulé du gène du facteur mitogène.

16. Procédé selon la revendication 15, selon lequel l'une des amorces est l'oligonucléotide de la revendication 9 ou 10 et l'autre amorce est l'oligonucléotide de la revendication 13 ou 14.

17. Procédé selon la revendication 15 ou 16, selon lequel les amorces ont les séquences SEQ ID NOs: 3 et 4, respectivement.

18. Procédé selon l'une quelconque des revendications 15 à 17, selon lequel les séquences nucléotidiques qui servent de matrice d'ADN sont extraites à partir de streptocoques du groupe A.

19. Procédé de microdétection pour les streptocoques du groupe A, comprenant l'étape consistant à détecter le gène du facteur mitogène par le procédé selon l'une quelconque des revendications 15 à 18.

20. Procédé selon la revendication 19, comprenant en outre l'étape consistant à pré-traiter les bactéries de test avec une protéase pour inactiver l'activité DNase.
